# FASCICULE DE BREVET EUROPEEN

(11) **EP 0 497 680 B1**
(45) Date de publication et mention de la délivrance du brevet: **27.09.1995**
(21) Numéro de dépôt: 92400207.4
(22) Date de dépôt: 27.01.1992
(51) Int. Cl.: C07C 43/04, C07C 41/42, C07C 31/08, C07C 29/80

(54) **Procédé de séparation d'éthyl tertiobutyléther et d'éthanol**
Verfahren zur Trennung von Ethyl-Tert-Butylether und Ethanol
Process for the separation of ethyl tert-butyl ether and ethanol

(30) Priorité: 30.01.1991 FR 9101132
(43) Date de publication de la demande: 05.08.1992
(73) Titulaire: INSTITUT FRANCAIS DU PETROLE, 92502 Rueil-Malmaison (FR)
(72) Inventeur: Pucci, Annick, F-78290 Croissy sur Seine (FR); Mikitenko, Paul, F-78590 Noisy le Roi (FR); Zuliani, Massimo, F-92500 Rueil Malmaison (FR)

(56) Documents cités:
- US-A- 2 721 222
- US-A- 3 940 450
- US-A- 4 440 963
- US-A- 5 015 783

## Description

On sait que l'éthyl tertiobutyl éther, tout comme le méthyl tertiobutyl éther (en abrégé MTBE), peut être utilisé comme additif à haut indice d'octane pour les essences sans plomb ou à teneur en plomb réduite. On peut prévoir d'ajouter l'ETBE aux essences à des concentrations allant par exemple jusqu'à environ 15 % en volume.

Un procédé de fabrication du MTBE consiste à réaliser une réaction d'addition de méthanol sur de l'isobutène, par exemple contenu dans une coupe C₄ de vapocraquage ou de craquage catalytique. Après réaction, le méthanol résiduel est en général séparé par distillation azéotropique avec les hydrocarbures en C₄, ce qui permet d'obtenir assez aisément le MTBE avec un degré de pureté convenable pour être additionné aux essences. Une telle technique a été décrite par exemple dans le brevet européen EP-B-78422 ou le brevet US-A-4 302 298.

La fabrication de l'ETBE peut être réalisée par un procédé analogue, dans lequel l'éthanol remplace le méthanol. Un tel procédé est décrit par exemple dans "l'ETBE, un avenir pour l'éthanol" de A. FORESTIERE, B. TORCK et G. PLUCHE, communication faite à la Conférence sur la Biomasse pour l'Energie et l'Industrie, Lisbonne, 9-13 Octobre 1989 et dans "MTBE/ETBE, an Incentive Flexibility for Refiners" par A. FORESTIERE et coll., communication faite à la "Conference on Oxygenated Fuels in Europe", Londres, 22-23 Mai 1990.

Cependant, par un tel procédé, contrairement au cas du MTBE, après élimination des hydrocarbures en C₄, la quasi-totalité de l'éthanol résiduel se retrouve en mélange avec l'ETBE produit. L'existence d'un azéotrope éthanol-ETBE à 21 % en poids d'éthanol à la pression atmosphérique, bouillant à 66,6 °C, rend difficile la séparation de l'ETBE avec un degré de pureté suffisant pour satisfaire les spécifications concernant la teneur en éthanol des essences. Ainsi la teneur en éthanol de l'ETBE doit être généralement comprise entre 0,5 et 10 % en poids. Avantageusement, l'ETBE devra être purifié à moins de 2 % en poids d'éthanol pour être acheminé à la raffinerie.

Ainsi, pour que l'ETBE puisse concurrencer le MTBE comme additif améliorant l'indice d'octane des essences sans plomb, il était particulièrement souhaité de trouver un procédé de séparation économiquement attractif. C'est ce que l'invention propose.

Le brevet des Etats-Unis US-A-4 440 963 décrit un procédé de production d'ETBE dans lequel la séparation de l'ETBE à partir de l'effluent de réaction, contenant notamment outre l'ETBE, de l'isobutène non réagi et de l'éthanol non réagi, est effectuée par un fractionnement en présence de 2-méthyl pentane, qui permet de recueillir en tête l'éthanol non réagi sous forme d'un azéotrope avec le 2-méthyl pentane, cet effluent de tête contenant aussi l'isobutène non réagi, et qui permet de recueillir en fond l'ETBE.

L'invention a donc pour objet un procédé de séparation de l'ETBE à partir de mélanges qu'il forme avec l'éthanol, et plus particulièrement à partir des mélanges ETBE-éthanol issus de la réaction de l'éthanol sur une coupe C₄ de vapocraquage ou de craquage catalytique.

L'invention a également pour objet un procédé de fabrication de l'ETBE incluant une telle opération de séparation et dans lequel l'éthanol est recyclé au réacteur d'éthérification.

Le procédé de séparation de l'ETBE de l'invention s'adresse en général aux mélanges constitués essentiellement d'éthanol et d'ETBE en proportions variées, et plus particulièrement aux mélanges issus de la réaction d'addition d'éthanol sur une coupe C₄, qui contiennent en général d'environ 5 à 50 %, le plus souvent de 10 à 30 %, en poids d'éthanol. Ils peuvent contenir en outre de très faibles proportions d'autres constituants, essentiellement des dimères de l'isobutène, tels que les triméthylpentènes, de l'alcool butylique tertiaire, du diéthyléther et des hydrocarbures en C₅.

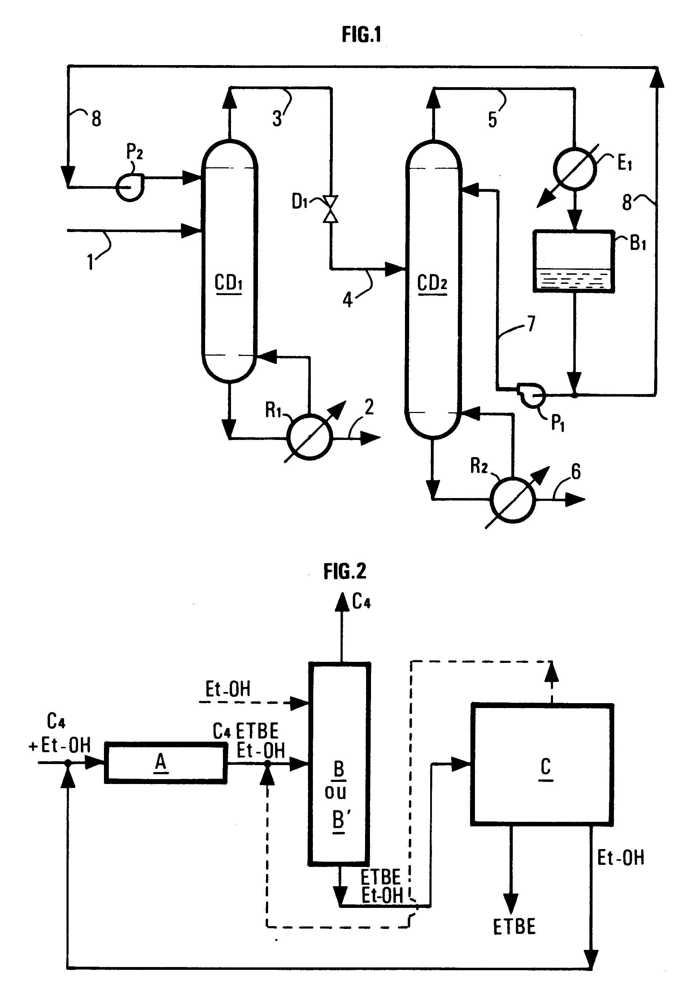

Le procédé de séparation d'ETBE de l'invention, qui globalement met en oeuvre deux distillations successives à des pressions différentes, est décrit plus en détail ci-après en relation avec la Figure 1 annexée. Les pressions indiquées dans la présente description sont des pressions absolues.

La charge consistant en le mélange contenant de l'éthanol et de l'ETBE à séparer est envoyée par la ligne 1 dans une première colonne de distillation CD₁ sous une pression p₁ supérieure ou égale à 1 bar, par exemple de 2,5 à 15 bars. Chauffée par un rebouilleur R₁, cette colonne opère entre une température de fond allant en général de 78 à 180 °C et une température de tête allant en général de 67 à 160 °C. La charge est introduite préférentiellement au point de bulle à la pression considérée, au plateau ayant la composition la plus voisine possible de celle de ladite charge, par exemple dans la partie supérieure de la colonne pour des compositions voisines de 20 % en poids d'éthanol (qui, dans la fabrication de l'ETBE, correspondent à un large excès d'éthanol par rapport à la quantité d'isobutène présente à l'entrée du réacteur d'éthérification).

Le résidu de la distillation, sortant en fond de colonne par la ligne 2 est constitué d'ETBE purifié.

En tête de colonne, le distillat sortant par la ligne 3 a une composition voisine de la composition de l'azéotrope à la pression p₁. Par exemple pour une pression p₁ de 5 bars, la composition du distillat est voisine de 30 % en poids d'éthanol pour 70 % en poids d'ETBE.

Ce distillat est détendu dans la vanne de détente D₁ à une pression p₂, en général inférieure à la pression p₁ d'une valeur Δp de 0,5 à 12 bars, plus particulièrement de 2 à 10 bars ; p₂ est alors en général de 0,5 à 10 bars. Après détente, le distillat est introduit par la ligne 4 à la partie supérieure d'une seconde colonne de distillation CD₂, fonctionnant à la pression p₂. Chauffée par le rebouilleur R₂, cette colonne opère entre une température de fond allant en général de 60 à 150 °C et une température de tête allant en général de 50 à 130 °C.

En tête de la colonne CD₂, on récupère par la ligne 5 un distillat ayant une composition voisine de l'azéotrope sous la pression p₂, contenant donc une proportion d'éthanol inférieure à celle de l'alimentation (distillat de première colonne). Pour une pression p₂ de 1 bar, la composition du distillat peut être d'environ 21 % en poids d'éthanol et 79 % en poids d'ETBE.

En fond de colonne, par la ligne 6, on récupère un résidu constitué d'éthanol purifié.

Le distillat sortant par la ligne 5 est amené à un échangeur de chaleur E₁ où il libère de la chaleur en se condensant. Le liquide est recueilli dans le ballon B₁.

A partir du ballon B₁, une partie du liquide est pompée par la pompe P₁ et renvoyée par la ligne 7 vers la tête de la colonne CD₂, dans laquelle elle agit comme reflux ; la proportion de liquide ainsi renvoyée est déterminée en fonction de la pureté que l'on souhaite atteindre pour l'éthanol recueilli en fond de colonne. Le reste du liquide est recyclé par la ligne 8 et la pompe P₂ vers la tête de la première colonne CD₁, où il fait office de reflux liquide.

Selon les conditions opératoires mises en oeuvre, l'ETBE obtenu comme résidu en fond de première colonne peut avoir un degré de pureté d'au moins environ 98 % en moles (c'est-à-dire au moins environ 99 % en poids).

On peut ajuster les conditions opératoires de manière à obtenir un produit de teneur en éthanol plus réduite, par exemple aussi faible que 1000 ppm en moles. La pureté de l'ETBE atteint alors 99,95 % en poids.

Le degré de pureté de l'ETBE obtenu dépend notamment du reflux mis en oeuvre en tête de colonne CD₁, comprenant d'une part le recyclage du distillat de la colonne CD₂ par la ligne 8, et d'autre part un éventuel reflux liquide provenant des vapeurs de tête de la ligne 3, ce reflux éventuel n'étant pas représenté sur la Figure 1.

Le résidu de deuxième colonne peut présenter une teneur en éthanol d'au moins 96 % en moles (c'est-à-dire au moins environ 92 % en poids).

On peut encore ajuster les conditions opératoires de manière que le produit contienne aussi peu que 1000 ppm en moles d'ETBE. La pureté de l'éthanol atteint alors 99,8 % en poids.

Parmi les impuretés éventuellement contenues dans la charge à traiter par le procédé de séparation de l'invention, les dimères de l'isobutène (c'est-à-dire essentiellement les triméthylpentènes) sont recueillis avec l'ETBE en fond de première colonne ; l'alcool butylique tertiaire est recueilli avec l'éthanol en fond de deuxième colonne ; le diéthyléther et les hydrocarbures en C₅ sortent en tête de première colonne ; ils n'affectent ni les températures opératoires, ni la pureté de l'ETBE produit.

Le procédé de l'invention présente l'avantage d'une grande simplicité et d'un coût réduit en énergie. Ainsi, la dépense d'énergie calorifique pour traiter une charge contenant par exemple 80 % en poids d'ETBE et 20 % en poids d'éthanol ne dépasse pas en général 600 kcal par kilogramme de charge traitée, pour des produits obtenus avec des puretés de 99,9 % en moles.

Le procédé de séparation de l'ETBE de l'invention, tel qu'il a été décrit ci-dessus, peut-être avantageusement intégré dans un procédé complet de fabrication d'ETBE par éthérification au moyen d'éthanol de l'isobutène contenu dans une coupe C₄ de vapocraquage ou de craquage catalytique.

Le procédé de fabrication d'ETBE, décrit en liaison avec le schéma de la Figure 2 annexée, comprend alors les étapes suivantes : dans une zone A, on met en contact dans des conditions de réaction de l'éthanol et une coupe C₄ de vapocraquage ou de craquage catalytique ; le produit issu de la zone réactionnelle A contient principalement de l'ETBE, de l'éthanol, des hydrocarbures en C₄ autres que l'isobutène, et à titre d'impuretés, de l'isobutène non réagi, des dimères de l'isobutène (essentiellement des triméthylpentènes), de l'alcool butylique tertiaire, du diéthyléther et des hydrocarbures en C₅ (pentanes et pentènes) déjà contenus dans la coupe C₄ initiale. Ce produit est envoyé dans une zone de distillation B, où sont séparés en tête les hydrocarbures en C₄ très appauvris en isobutène et, en fond, un mélange d'ETBE et d'éthanol contenant les autres impuretés. Si l'on souhaite une élimination plus complète de l'isobutène, on peut mettre en oeuvre, au lieu d'une simple distillation B, une distillation réactive B', mettant en jeu un appoint d'éthanol, comme indiqué en pointillés sur la Figure 2. Dans tous les cas, le mélange d'ETBE et d'éthanol, recueilli en fond de zone B est envoyé dans une zone C, où est réalisé le procédé de séparation de l'invention.

Il est particulièrement intéressant de noter que, en général, à l'issue du traitement réalisé dans la zone B, le mélange contenant principalement de l'ETBE et de l'éthanol est obtenu sous une pression suffisante pour pouvoir être introduit directement dans la première colonne de distillation de la zone de séparation C.

A partir de la zone C, l'éthanol purifié recueilli est avantageusement recyclé comme appoint vers la zone de réaction A et/ou vers la zone de distillation réactive B'.

Par ailleurs, les impuretés légères, consistant en traces de diéthyléther et d'hydrocarbures en C₅, peuvent être éliminées par une purge effectuée en tête de première colonne ou en tête de deuxième colonne et recyclées vers l'entrée de la zone de distillation B, comme indiqué schématiquement en traits pointillés sur la Figure 2. Comme déjà indiqué plus haut, parmi les impuretés, les dimères de l'isobutène sont recueillis avec l'ETBE et l'alcool butylique tertiaire avec l'éthanol.

Les exemples suivants illustrent l'invention.

### EXEMPLE 1

On a réalisé la séparation d'un mélange ETBE-éthanol de manière à obtenir l'ETBE à un degré de pureté de l'ordre de 99 % en poids.

La charge est constituée de 80 % en poids d'ETBE et de 20 % en poids d'éthanol. Elle est introduite, sur le 6ème plateau, sous 5 bars et à 119 °C sous un débit de 1,5 kg/heure dans une première colonne de distillation opérant à 5 bars.

Cette colonne, en acier inoxydable, d'un diamètre de 5 cm, comporte 16 plateaux perforés à déversoir espacés de 5 cm.

Elle est munie d'un bouilleur chauffé électriquement. Toute la colonne est isolée thermiquement de manière à éviter les déperditions de calories. La température de la colonne s'étage entre 132 °C en fond et 118 °C en tête.

Le distillat contenant 32 % en poids d'éthanol et 68 % en poids d'ETBE est détendu à 1 bar. Sous un débit de 3,39 kg/heure, il alimente sur le 6ème plateau une seconde colonne opérant sous 1 bar.
Cette seconde colonne est une colonne en verre de 50 mm de diamètre, adiabatique, munie de 20 plateaux perforés à déversoir, d'un bouilleur chauffé électriquement, d'un condenseur à eau et d'un ballon de reflux.

La température de la colonne s'étage entre 75 °C en fond et 67 °C en tête.

Le distillat contenant 74 % en poids d'ETBE et 26 % en poids d'éthanol est condensé. Une fraction du condensat est renvoyée sous un débit de 3,69 kg/heure en tête de seconde colonne, à titre de reflux. Le reste du condensat, sous un débit de 3,07 kg/heure est recyclé en tête de la première colonne.

On recueille :
. en fond de première colonne, sous un débit de 1,18 kg/heure, un produit contenant 99,1 % en poids d'ETBE et 0,9 % en poids d'éthanol,
. et en fond de seconde colonne, sous un débit de 0,314 kg/heure, un produit contenant 93,6 % en poids d'éthanol et 6,4 % en poids d'ETBE.

Le procédé mis en oeuvre dans cet exemple nécessite un apport d'énergie d'environ 450 kcal/h au rebouilleur R₁ et d'environ 180 kcal/heure au rebouilleur R₂.

### EXEMPLE 2

On a réalisé la séparation d'un mélange ETBE-éthanol de manière à obtenir chacun des deux constituants avec une teneur résiduelle en l'autre d'environ 1000 ppm, en moles.

La même charge que dans l'exemple 1 est introduite sous un débit de 1,5 kg/heure dans la première colonne de distillation, munie de 30 plateaux, sous une pression de 5 bars, qui opère entre une température de fond de 135 °C et une température de tête de 118 °C.

Le distillat contenant 70 % en poids d'ETBE et 30 % en poids d'éthanol, après détente à 1 bar, est envoyé sous un débit de 3,93 kg/heure à la seconde colonne travaillant sous 1 bar. La température de celle-ci s'étage entre 78 °C en fond et 67 °C en tête.

Le distillat, qui contient 75 % en poids d'ETBE ET 25 % en poids d'éthanol, est condensé. Une fraction du condensat est renvoyée vers la tête de la seconde colonne sous un débit de 4,35 kg/heure, le reste étant recyclé, sous un débit de 3,63 kg/heure vers la tête de la première colonne.

On recueille :
. en fond de première colonne, sous un débit de 1,2 kg/heure, un produit contenant 99,95 % en poids d'ETBE ET 0,05 % en poids d'éthanol,
. et en fond de seconde colonne, sous un débit de 0,3 kg/heure, un produit contenant 99,8 % en poids d'éthanol et 0,2 % en poids d'ETBE.

Le procédé mis en oeuvre dans cet exemple nécessite un apport d'énergie d'environ 510 kcal/heure au premier rebouilleur R₁ et d'environ 330 kcal/heure au second rebouilleur R₂.

## Revendications

1. Procédé ce séparation de l'éthyl tertiobutyl éther à partir des mélanges qu'il forme avec l'éthanol, caractérisé en ce que :
a) on introduit une charge constituée essentiellement d'un mélange d'éthyl tertiobutyl éther et d'éthanol dans une première colonne de distillation opérant sous une pression p₁ supérieure ou égale à 1 bar, et à une température allant d'une température de fond de 78 à 180 °C à une température ce tête de 67 à 160 °C ;
b) le distillat sortant en tête de ladite première colonne avec une composition voisine de la composition de l'azéotrope à ladite pression p₁, est détendu à une pression p₂ inférieure à p₁ d'une valeur Δp de 0,5 à 12 bars, p₂ étant alors de 0,5 à 10 bars, avant d'être introduit dans une seconde colonne de distillation opérant à ladite pression p₂ et à une température allant d'une température de fond de 60 à 150 °C à une température de tête de 50 à 130 °C ;
c) le distillat sortant en tête de ladite seconde colonne avec une composition voisine de la composition de l'azéotrope à ladite pression p₂ est condensé en une phase liquide, en partie renvoyée vers la tête de la seconde colonne et en partie recyclée vers la tête de la première colonne ;
d) on recueille l'ETBE purifié en fond de première colonne ; et
e) on recueille l'éthanol purifié en fond de deuxième colonne.

2. Procédé selon la revendication 1, caractérisé en ce que la charge comprend de 5 à 50 % en poids d'éthanol.

3. Procédé selon la revendication 1, caractérisé en ce que la charge comprend de 10 à 30 % en poids d'éthanol.

4. Procédé selon l'une des revendications 1 à 3, caractérisé en ce que la pression p₁ est de 2,5 à 15 bars et la pression p₂ est inférieure à la pression p₁ d'une valeur Δp de 2 à 10 bars.

5. Procédé selon l'une des revendications 1 à 4, caractérisé en ce que la charge provient d'une fabrication d'ETBE par éthérification par de l'éthanol de l'isobutène contenu dans une coupe C₄ de vapocraquage ou de craquage catalytique et contient, outre l'ETBE et l'éthanol, de très faibles proportions de dimères de l'isobutène, d'alcool butylique tertiaire, de diéthyléther et d'hydrocarbures en C₅, lesdits dimères de l'isobutène étant recueillis en fond de première colonne en mélange avec l'ETBE, ledit alcool butylique tertiaire étant recueilli en fond de seconde colonne en mélange avec l'éthanol, ledit diéthyléther et lesdits hydrocarbures en C₅ sortant en tête de première colonne.

6. Procédé selon la revendication 5, caractérisé en ce que la charge renferme environ 80 % en poids d'ETBE et environ 20 % en poids d'éthanol.

7. Procédé de fabrication d'éthyl tertiobutyl éther par éthérification par de l'éthanol de l'isobutène contenu dans une coupe C₄ de vapocraquage ou de craquage catalytique, dans lequel, dans une zone de réaction A, on met en contact dans des conditions réactionnelles de l'éthanol et ladite coupe C₄ ; le produit issu de ladite zone de réaction A, contenant principalement de l'ETBE, de l'éthanol, des hydrocarbures en C₄ autres que l'isobutène, et, à titre d'impuretés, de l'isobutène non réagi, des dimères de l'isobutène, de l'alcool butylique tertiaire, du diéthyléther et des hydrocarbures en C₅, est envoyé dans une zone de distillation B où sont séparés en tête les hydrocarbures en C₄ incluant l'isobutène non réagi et en fond un mélange d'ETBE et d'éthanol contenant les autres impuretés ; on envoie ledit mélange dans une zone de séparation C ; ledit procédé étant caractérisé en ce que, dans ladite zone de séparation C, on met en oeuvre un procédé selon l'une des revendications 1 à 6.

8. Procédé selon la revendication 7, caractérisé en ce que ladite zone de distillation B est remplacée par une zone de distillation réactive B', dans laquelle on introduit de l'éthanol pour éliminer l'isobutène non réagi.

9. Procédé selon l'une des revendications 7 et 8, caractérisé en ce que l'éthanol purifié issu de la zone de séparation C est recyclé à l'entrée de la zone de réaction A et/ou vers la zone de distillation B'.

10. Procédé selon l'une des revendications 7 à 9, caractérisé en ce que l'on effectue une purge sur le distillat de première colonne ou sur le distillat de deuxième colonne de la zone de séparation C, ladite purge étant recyclée vers l'entrée de la zone de distillation B (ou B').

## Claims

1. Process for the separation of tert. butyl ethyl ether from mixtures which it forms with ethanol, characterized in that:
a) a charge essentially constituted by a mixture of tert. butyl ethyl ether and ethanol is introduced into a first distillation column operating under a pressure p₁ equal to or higher than 1 bar and at a temperature ranging between a bottom temperature of 78 to 180°C and a head temperature of 67 to 160°C,
b) the distillate leaving the head of said first column and having a composition close to that of the azeotrope at said pressure p₁ is expanded to a pressure p₂ lower than p₁ by a value Δp of 0.5 to 12 bars, p₂ then being 0.5 to 10 bars, before being introduced into a second distillation column operating at said pressure p₂ and at a temperature ranging between a bottom temperature of 60 to 150°C and a head temperature of 50 to 130°C,
c) the distillate leaving the head of said second column and having a composition close to the composition of the azeotrope at said pressure p₂ is condensed to a liquid phase, partly supplied to the head of the second column and partly recycled to the head of the first column,
d) purified ETBE is collected at the bottom of the first column and
e) purified ethanol is collected at the bottom of the second column.

2. Process according to claim 1, characterized in that the charge comprises 5 to 50% by weight ethanol.

3. Process according to claim 1, characterized in that the charge comprises 10 to 30% by weight ethanol.

4. Process according to any one of claims 1 to 3, characterized in that the pressure p₁ is 2.5 to 15 bars and the pressure p₂ is lower than the pressure p₁ by a value Δp of 2 to 10 bars.

5. Process according to any one of claims 1 to 4, characterized in that the charge results from a production of ETBE by etherification with ethanol of isobutylene contained in a steam or catalytic cracking C₄ fraction and contains, apart from ETBE and ethanol, very small proportions of dimers of isobutylene, tert. butyl alcohol diethyl ether and C₅ hydrocarbons, said isobutylene dimers being collected at the bottom of the first column mixed with ETBE, the said tert. butyl alcohol being collected at the bottom of the second column mixed with ethanol, said diethyl ether and said C₅ hydrocarbons passing out of the head of the first column.

6. Process according to claim 5, characterized in that the charge contains approximately 80% by weight ETBE and approximately 20% by weight ethanol.

7. Process for the production of tert. butyl ethyl ether by etherification with ethanol of isobutylene contained in a catalytic or steam cracking C₄ fraction, in which, in a reaction zone A, contacting takes place, under reaction conditions, between ethanol and said C₄ fraction, whereby the product from the said reaction zone A mainly containing ETBE, ethanol, C₄ hydrocarbons other than isobutylene and, as impurities, unreacted isobutylene, isobutylene dimers, tert. butyl alcohol, diethyl ether and C₅ hydrocarbons is supplied to a distillation zone B, where separation takes place at the head of the C₄ hydrocarbons including the unreacted isobutylene and at the bottom a mixture of ETBE and ethanol containing the other impurities, said mixture is supplied to a separation zone C ; said process being characterized in that in the said separation zone C is performed a process according to any one of the claims 1 to 6.

8. Process according to claim 7, characterized in that the distillation zone B is replaced by a reactive distillation zone B', into which is introduced the ethanol for eliminating the unreacted isobutylene.

9. Process according to one of claims 7 and 8, characterized in that the purified ethanol from the separation zone C is recycled to the intake of the reaction zone A and/or to the distillation zone B'.

10. Process according to any one of claims 7 to 9, characterized in that a purge is carried out on the distillate of the first column or on the distillate of the second column of the separation zone C, said purge being recycled to the intake of the distillation zone B or B'.

## Patentansprüche

1. Verfahren zur Abtrennung von Ethyl-tert.-butylether aus Gemischen, die er mit Ethanol bildet, dadurch gekennzeichnet, daß:
a) man eine im wesentlichen aus einer Mischung von Ethyl-tert.-butylether und Ethanol bestehende Charge in eine erste Destillationskolonne einführt, welche unter einem Druck p₁ von höher oder gleich 1 bar und bei einer Temperatur, welche von einer Bodentemperatur von 78 bis 180°C bis zu einer Kopftemperatur von 67 bis 160°C reicht, arbeitet;
b) das am Kopf der ersten Kolonne austretende Destillat mit einer Zusammensetzung nahe der Azeotropzusammensetzung beim Druck p₁ auf einen Druck p₂, der um einen Wert Δp von 0,5 bis 12 bar unter p₁ liegt, entspannt wird, wobei p₂ dann 0,5 bis 10 bar beträgt, bevor es in eine zweite Destillationskolonne beim Druck p₂ und bei einer Temperatur, welche von einer Bodentemperatur von 60 bis 150°C bis zu einer Kopftemperatur von 50 bis 130°C reicht, eingeführt wird;
c) das aus dem Kopf der zweiten Kolonne austretende Destillat mit einer Zusammensetzung nahe der Azeotropzusammensetzung beim Druck p₂ zu einer flüssigen Phase kondensiert, teilweise zum Kopf der zweiten Kolonne und teilweise zum Kopf der ersten Kolonne zurückgeführt wird;
d) man den gereinigten ETBE am Boden der ersten Kolonne gewinnt; und
e) man gereinigtes Ethanol am Boden der zweiten Kolonne gewinnt.

2. Verfahren nach Anspruch 1, dadurch gekennzeichnet, daß die Charge 5 bis 50 Gew.-% Ethanol umfaßt.

3. Verfahren nach Anspruch 1, dadurch gekennzeichnet, daß die Charge 10 bis 30 Gew.-% Ethanol umfaßt.

4. Verfahren nach einem der Ansprüche 1 bis 3, dadurch gekennzeichnet, daß der Druck p₁ 2,5 bis 15 bar beträgt und der Druck p₂ um einen Wert Δp von 2 bis 10 bar unter dem Druck p₁ liegt.

5. Verfahren nach einem der Patentansprüche 1 bis 4, dadurch gekennzeichnet, daß die Charge aus einer ETBE-Herstellung durch Veretherung mit Ethanol von Isobuten stammt, das in einer C₄-Fraktion aus dem Dampfcracken oder dem katalytischen Cracken enthalten ist und neben ETBE und Ethanol sehr geringe Mengen von Isobutendimeren, tert.-Butylalkohol, Diethylether und C₅-Kohlenwasserstoffen enthält, wobei die Isobutendimere vom Boden der ersten Kolonne im Gemisch mit ETBE gewonnen werden, der tert.-Butylalkohol vom Boden der zweiten Kolonne im Gemisch mit Ethanol gewonnen wird, der Diethylether und die C₅-Kohlenwasserstoffe am Kopf der Kolonne austreten.

6. Verfahren nach Anspruch 5, dadurch gekennzeichnet, daß die Charge etwa 80 Gew.-% ETBE und etwa 20 Gew.-% Ethanol umfaßt.

7. Verfahren zur Herstellung von Ethyl-tert.-butylether durch Veretherung mit Ethanol von Isobuten, das in einer C₄-Fraktion aus dem Dampfcracken oder dem katalytischen Cracken enthalten ist, worin in einer ersten Reaktionszone A man unter Umsetzungsbedingungen Ethanol und die C₄-Fraktion in Kontakt bringt; das aus der Reaktionszone A stammende Produkt, das hauptsächlich ETBE, Ethanol, von Isobuten verschiedene C₄-Kohlenwasserstoffe und als Verunreinigungen nicht umgesetztes Isobuten, Dimere von Isobuten, tert.-Butylalkohol, Diethylether und C₅-Kohlenwasserstoffe enthält, in eine Destillationszone B eingeführt wird, wo am Kopf die C₄-Kohlenwasserstoffe, einschließlich nicht umgesetztes Isobuten, und am Boden eine Mischung von ETBE und Ethanol, welche die anderen Verunreinigungen enthält, abgetrennt werden; man die Mischung in eine Abtrennzone C einführt, wobei das Verfahren dadurch gekennzeichnet ist, daß man in der Abtrennzone C ein Verfahren nach einem der Ansprüche 1 bis 6 durchführt.

8. Verfahren nach Anspruch 7, dadurch gekennzeichnet, daß die Destillationszone B durch eine reaktive Destillationszone B' ersetzt wird, in welche man Ethanol einführt, um nicht umgesetztes Isobuten zu beseitigen.

9. Verfahren nach einem der Ansprüche 7 und 8, dadurch gekennzeichnet, daß aus der Abtrennzone C stammendes, gereinigtes Ethanol zum Eingang der Reaktionszone A und/oder zur Destillationszone B' zurückgeführt wird.

10. Verfahren nach einem der Ansprüche 7 bis 9, dadurch gekennzeichnet, daß man am Destillat der ersten Kolonne oder am Destillat der zweiten Kolonne der Abtrennzone C eine Spülung bewirkt, wobei die Spülung zum Eingang der Destillationszone B (oder B') zurückgeführt wird.
